# EUROPEAN PATENT SPECIFICATION

(11) **EP 1 592 397 B1**
(45) Date of publication and mention of the grant of the patent: **31.03.2010**
(21) Application number: 04704302.1
(22) Date of filing: 22.01.2004
(51) Int. Cl.: A61Q 19/02, A61K 8/34, A61K 8/49

(54) **IMPROVED FORMULATION FOR SKIN-LIGHTENING AGENTS**
VERBESSERTE FORMULIERUNG FÜR HAUTBLEICHMITTEL
FORMULATION AMELIOREE D'AGENTS ECLAIRCISSANT DE LA PEAU

(30) Priority: 03.02.2003 US 444496 P
(43) Date of publication of application: 09.11.2005
(73) Proprietor: Warner-Lambert Company LLC, New York, NY 10017 (US)
(72) Inventor: NAIR, Xina, St. James, NY 11780 (US)
(74) Representative: Motion, Keith Robert
(86) International application number: PCT/IB2004/000240
(87) International publication number: WO 2004/069221

(56) References cited:
- US-A- 5 824 326
- US-A- 6 132 740
- US-A1- 2002 035 046
- US-A1- 2002 155 075
- US-A1- 2002 161 041
- E. SQUILLANTE ET AL: "Codiffusion of propylene glycol and dimethylisosorbide in hairless mouse skin" EUROPEAN JOURNAL OF PMARMACEUTICS AND BIOPHARMACEUTICS, vol. 46, 1998, pages 265-271, XP004257000

## Description

### Field of the Invention

The present invention is directed to a topical formulation suitable for administering depigmenting agents.

### Summary of the Related Art

In humans, skin color (pigmentation) arises from a complex series of cellular processes that are carried out within a group of cells know as melanocytes. Melanocytes are located in the lower part of the epidermis and their function is to synthesize a pigment, melanin, which protects the body from the damaging effects of ultraviolet radiation.

The mechanism by which skin pigmentation is formed, melanogenesis, involves the following main steps: Tyrosine → L-Dopa → Dopaquinone Dopachrome → Melanin. The first two reactions in this series am catalyzed by the enzyme, tyrosinase. The activity of tyrosinase is promoted by the action of α-melanocyte stimulating hormone and UV rays.

Typically, melanogenesis leads to a darker skin tone (i.e. a tan). However, melanogenesis can lead to undesirable pigmentation patterns as well. Examples of such undesirable pigmentation include age spots, liver spots, etc. Such inappropriate pigmentation has lead to research to find compounds that will inhibit melanogenesis. One of the targets of this research is the inhibition of tyrosinase, the enzyme which catalyses the initial step in the generation of melanin.

US 2002/0155075 discloses the use of 4-(2,4-dihydroxy-phenyl)cyclohexanol as a skin lightening agent and formulations containing such compound.

US 5,824,326 discloses compositions containing ferulic acid or its C₁-C₃₀ alcohol esters and dimethyl isosorbide in a pharmaceutically acceptable carrier.

United States Patent No. 6,132,740 discloses a class of tyrosinase inhibitors that may be used as skin lighteners. All of the compounds of the '740 patent are 4-cycloalkyl resorcinols. The depigmenting activity of 4-cyclopentyl resorcinol and 4-cyclohexyl resorcinol is depicted in an animal model in the '740 patent (see column 10, lines 36-44). The formulation utilized for these tests was a 70:30 admixture of ethanol and propylene glycol with five (5) % of either 4-cyclohexyl resorcinol or 4-cyclopentyl resorcinol.

While the formulation of the '740 patent was efficacious as a skin lightening agent, there is always room for improvements. Such improvements include decreasing the amount of active agent that is required to achieve the therapeutic effect.

### SUMMARY OF THE INVENTION

In accordance with the present invention, a new topical formulation for skin lightening agents, such as 4-cycloalkyl resorcinol, has been discovered. This new formulation enhances the efficacy of the skin-lightening agent. It allows the physician, or consumer, to utilize a lower dose of the skin-lightening agent (i.e. to utilize a lower concentration of the skin lightening agent).

The new formulation contains a skin-lightening agent in combination with a carrier. The carrier contains at least one hydroxyl solvent and at least one co-solvent. Examples of suitable hydroxyl solvents include glycols, lower alkanols, or water. Examples of suitable co-solvents include a dianhydrohexitol, a cyclic amide, cyclic carbonate, cyclic carbamate, or a lower alkyl ester of a C₁₀-C₂₀ fatty acid.

Typically, the formulation will be a liquid. It will contain from about 0.1 to about 10% (wt/vol) of the skin-lightening agent. The formulation will also contain a carrier. The carrier may constitute up to 99.9 % (vol/vol) of the formulation, but at a minimum will constitute at least 80% (vol/vol) of the formulation. The carrier will comprise 5 v/v % of hexylene glycol, 40% v/v of propylene glycol, 15% v/v of dimethylisosorbide and 40% v/v of ethanol.

In a more specific embodiment, the skin-lightening agent is 4-cyclopentyl resorcinol, present in the quantity of 0.5 to 4% (wt/vol). The co-solvent is an isorbide present in the carrier in the quantity of about 10 to 20 % (vol/vol). The remainder of the carrier will be composed of an admixture of glycol and alcohol.

The formulation, which is typically a liquid, is used in a conventional manner for dermatologicals. The patient will apply the formulation to the area of the skin requiring lightening. The formulation may be applied from 1 to 4 times daily until the patient achieves the desired pigmentation tone.

Typically, the formulation will be packaged for retail distribution. Thus a further embodiment of the invention is directed to a kit containing the formulation, packaged in a manner suitable for retail distribution, and labeled in a manner which instructs the patient, or health care provider, how to use the formulation in order to lighten their skin.

### BRIEF DESCRIPTION OF THE DRAWING

The invention is described with reference to the following figures, which are presented for the purpose of illustration only and which are not intended to be limiting of the intention.

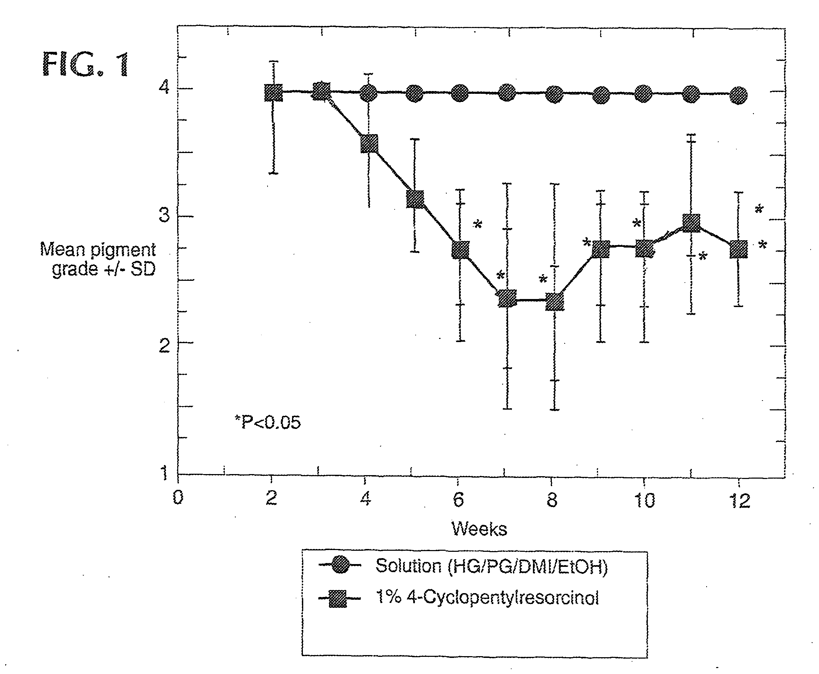

FIG. 1 is a graphic representation comparing the depigmenting effect on Yucatan swine of topically applied 1 % (wt/vol) 4-cyclopentylresorcinol in HG/PG/DMI/EtOH with the HG/PG/DMI/EtOH solution alone. The asterisks denote statistical significance from the solution alone using ANOVA and Kruskal-Wallis test (p<0.05).

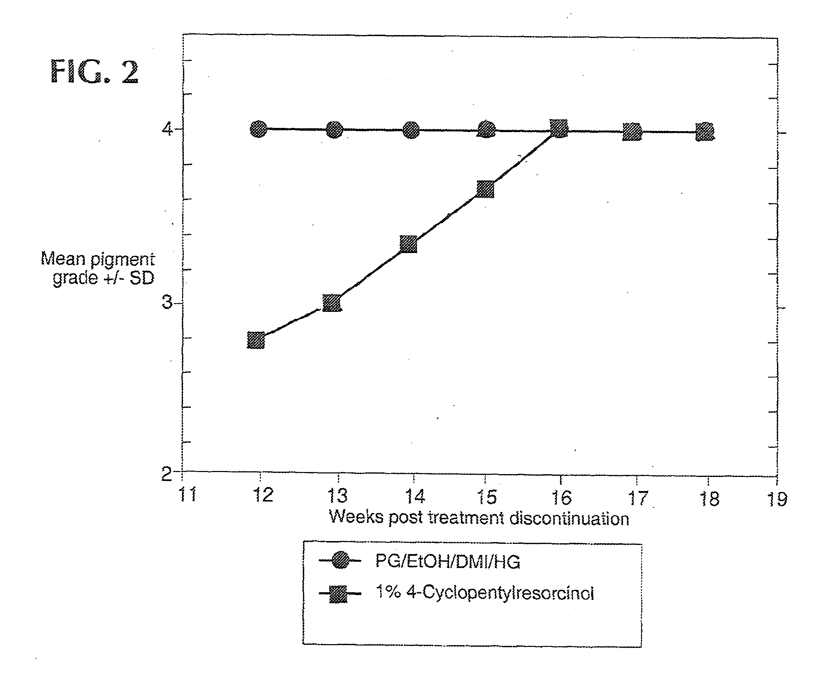

FIG. 2 is a graphic representation comparing the reversibility of depigmentation after treatment discontinuation for the HG/PG/DMI/EtOH solution alone to different concentrations of 4-cyclopentylresorcinol in the solution.

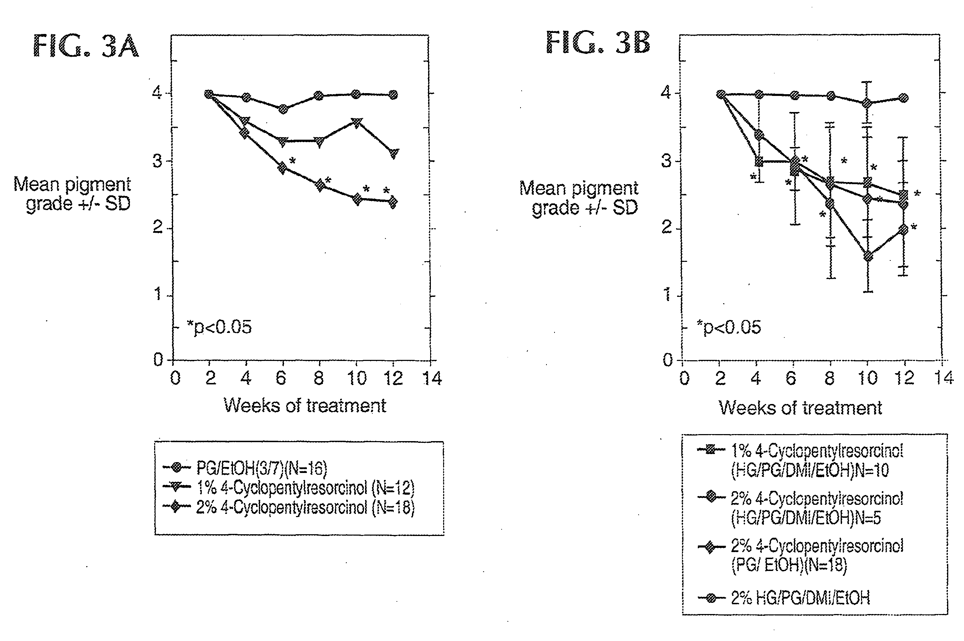

FIG. 3A is a graphic representation comparing the effect on the skin pigmentation of Yucatan swine of topical administration of 1 % (wt/vol) and 2% (wt/vol) 4-cyclopentylresorcinol in PG/EtOH. An asterisk denotes statistical difference from the respective vehicle controls.
FIG. 3B is a graphic representation comparing the effect on the skin pigmentation of Yucatan swine of topical administration of 1% (wt/vol) and 2% (wt/vol) 4-cyclopentylresorconol in HG/PG/DMI/EtOH to 2% (wt/vol) 4-cyclopentylresorcinol in PG/EtOH and to the HG/PG/DMI/EtOH vehicle. An asterisk denotes statistical difference from the vehicle control.

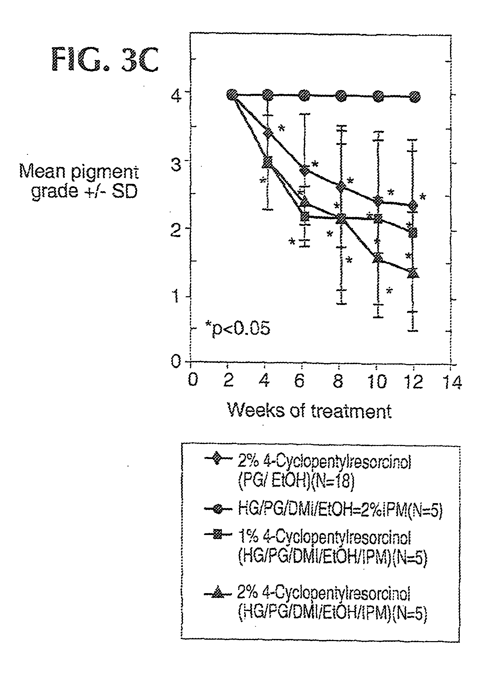

FIG. 3C is a graphic representation comparing the effect on the skin pigmentation of Yucatan swine of topical administration of 1% (wt/vol) and 2% (wt/vol) 4-cyclopentylresorcinol in HG/PG/DMI/EtOH/IPM to 2% (wt/vol) 4-cyclopentylresorcinol in PG/EtOH and to the HG/PG/DMI/EtOH plus 2% (wt/wt) IPM vehicle alone. An asterisk denotes statistical difference from the vehicle control.

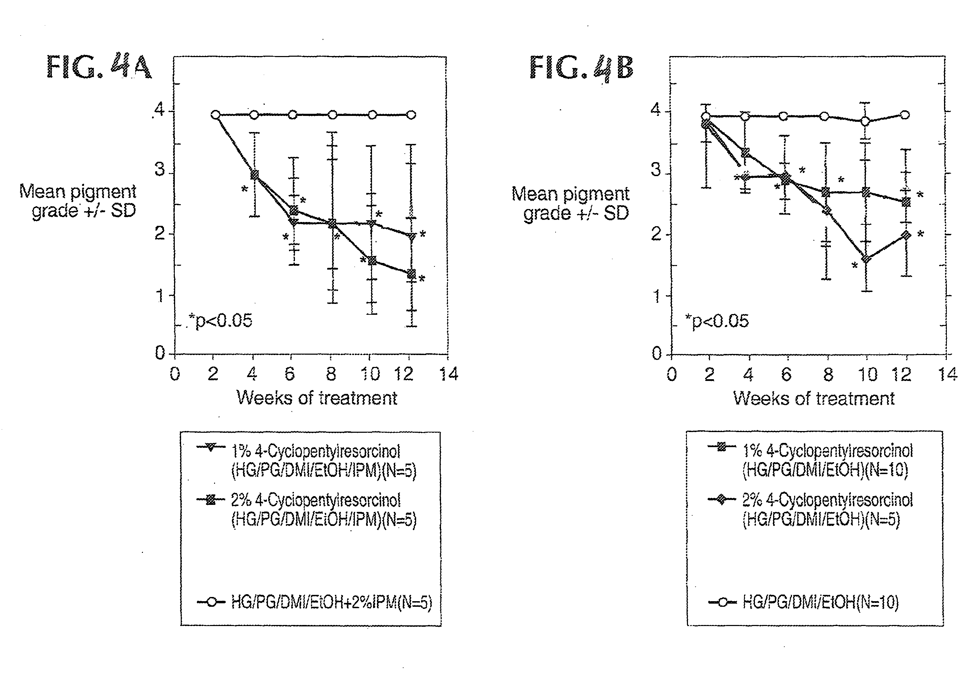

FIG. 4A is a graphic representation comparing the effect on the skin pigmentation of Yucatan swine of topical administration of 1% (wt/vol) and 2% (wt/vol) 4-cyclopentylresorcinol in an HG/PG/DMI/EtOH/IPM vehicle to the HG/PG/DMI/EtOH plus 2% (wt/wt) IPM vehicle alone. An asterisk denotes statistical difference from the vehicle control.
FIG. 4B is a graphic representation comparing the effect on the skin pigmentation of Yucatan swine of topical administration of 1% (wt/vol) and 2% (wt/vol) 4-cyclopentylresorcinol in a HG/PG/DMI/EtOH vehicle to the HG/PG/DMI/EtOH vehicle alone. An asterisk denotes statistical difference from the vehicle control.

### DETAILED DESCRIPTION

The headings below have been added to facilitate the review of this document by the reader. They should not be construed as limiting the disclosure or claims in any manner.

### A) DEFINITIONS

The issued U.S. patents, published patent applications, and references that are cited herein are hereby incorporated by reference to the same extent as if each were specifically and individually indicated to be incorporated by reference. Any inconsistency between these publications and the present disclosure shall be resolved in favor of the present disclosure.

The following terms are used herein according to the following definitions:

As used herein, the term "comprises" means "includes, but is not limited to."

The term "topical" or "topically" as used herein refers to the application of a formulation externally to the skin or a portion of the external surface of the body.

As used herein, the term "skin-lightening agent" encompasses an agent that increases the degree to which the skin, or a portion of the external surface of the body, is pale or white in color. Such an agent decreases the degree to which color or pigmentation is present or formed within the skin or a portion of the external surface of the body. In some embodiments, the effect achieved by a skin-lightening agent is achieved by preventing the formation of skin pigmentation. As used herein, this term is equivalent to the terms "active agent," "pigmentation-inhibiting agent," "lightening agent," "depigmenting agent" and "depigmentation agent."

The term "admixture" means two or more components mixed together resulting in a combination of the components. By way of nonlimiting example, one component is dissolved in another component.

The term "carrier" is used herein to refer to a pharmaceutically acceptable vehicle as described herein for the skin-lightening agent and/or other pharmacologically active agent. The carrier facilitates delivery of the skin-lightening agent to the target site in the skin of a subject without terminating the skin-lightening function of the agent. Any pharmaceutical excipient incorporated into the formulation should be considered part of the carrier.

The term "hydroxyl compound" refers to any compound having at least one hydroxyl group. For purposes of the invention, preferred hydroxyl compounds include, without limitation, glycols, lower alkanols, and water. Hydroxyl compound and hydroxyl solvent should be considered as synonoms.

The term "glycol" refers to a straight or branched chain hydrocarbon having at least two hydroxyl substituents. In some embodiments, the glycol has from about two to about twelve carbon atoms (C₂-C₁₂). As used herein, the term "glycol" also encompasses polymeric versions of the glycols described herein. For example, reference to a "C₂ glycol" is intended to include both ethylene glycol and polyethylene glycol.

The term "co-solvent" as used herein refers to a compound in which the skin-lightening agent is soluble. In some embodiments the co-solvent also serves additional functions, such as enhancing the activity of the skin-lightening agent.

A "fatty acid" as used herein refers to a long hydrocarbon chain that has a carboxyl group at one end. The hydrocarbon chain optionally includes one or more carbon-carbon double bonds.

As used herein, the term "derivative" means a compound that retains the underlying chemical structure of the original compound, but has been structurally modified on one or more functional groups. The term "derivative" includes, but is not limited to, compounds that can be readily converted into the original compound upon administration *in vivo, e.g.,* prodrug forms of the original compound.

As used herein, the term "analog" refers to a compound that retains the same core structure as the compound referred to, but is optionally structurally modified (such as by addition or substitution). The term "analog" is intended to encompass the core structure compound, as well as compounds with modified structures.

By way of example, the term "resorcinol analog" includes resorcinol (1,3-benzenediol) and substituted resorcinols, as defined herein. In some embodiments, the resorcinol analog is a 4-substituted resorcinol. As used herein, the term "resorcinol derivative" refers to a resorcinol analog wherein one or more hydroxyl groups of the resorcinol are derivatized, *e.g*., as ester, carbonate, carbamate, or ether derivatives. In some embodiments, the resorcinol derivative acts as a prodrug, which can be readily converted into the original resorcinol compound upon administration of the compound *in vivo,* such as upon topical application to the skin, such that the resorcinol derivative ultimately produces its biological (*e.g*., skin-lightening) effect. As used herein, reference to "a resorcinol" or "resorcinol compound" encompasses resorcinol analogs and resorcinol derivatives, as defined herein.

The term "pharmaceutically acceptable" is used herein to mean suitable for use in mammals. Pharmaceutically acceptable salts of a compound include acid and base addition salts thereof. Suitable acid addition salts are formed from acids that form non-toxic salts. Nonlimiting examples include hydrochloride, hydrobromide, hydroiodide, nitrate, sulfate, bisulfate, phosphate, acid phosphate, acetate, lactate, citrate, acid citrate, tartrate, bitartrate, succinate, maleate, fumarate, gluconate, saccharate, benzoate, methanesulfonate, ethanesulfonate, benzenesulfonate, p-toluenesulfonate and pamoate (*i.e*., 1,1-methylene-bis-(2-hydroxy-3-naphthoate)) salts. Suitable base salts are formed from bases which form non-toxic salts. Nonlimiting examples include aluminum, sodium, potassium, calcium, magnesium, zinc and diethanolammonium salts. For a review of suitable salts, *see, e.g.,* Berge et al, J. Pharm. Sci. 66:1-19 (1977) and Remington: The Science and Practice of Pharmacy, 20th Ed., ed. A. Gennaro, Lippincott Williams & Wilkins, 2000.

Pharmaceutically acceptable ester derivatives include those esters that retain, upon hydrolysis of the ester bond, the biological effectiveness and properties of the active carboxylic acid or alcohol component, and do not produce undesirable biological effects. For a description of pharmaceutically acceptable esters as prodrugs, see Bundgaard, E., ed., Design of Prodrugs, Elsevier Science Publishers, Amsterdam, 1985. Generally, ester formation is accomplished via conventional synthetic techniques. (*See, e.g.,* March's Advanced Organic Chemistry, 3rd Ed., John Wiley & Sons, New York p. 1157, 1985 and references cited therein, and Mark et al., Encyclopedia of Chemical Technology, John Wiley & Sons, New York, 1980.) Also contemplated within the scope of the invention are formulation components that individually comprise both a pharmaceutically acceptable salt moiety and a pharmaceutically acceptable ester moiety.

The term "lower alkanol" is intended to encompass all C₁-C₆ alkanols, including, without limitation, all straight chain or branched lower alkanols. These compounds may contain chiral centers and therefore may exist in different enantiomeric and diastereomeric forms. Any optical isomers, any regioisomers, and any stereoisomers of these compounds and mixtures thereof, can be used as the active agent in the formulation of the invention.

The term "about" is used herein to mean approximately, in the region of, roughly, or around. When the term "about" is used in conjunction with a numerical range, it modifies that range by extending the boundaries above and below the numerical values set forth. In general, the term "about" is used herein to modify a numerical value above and below the stated value by a variance of 20%.

The term "substituted" is used herein to refer to the addition of a functional group at a location on a compound. It means that one or more hydrogens of the designated moiety are replaced, provided that no atom's normal valency is exceeded, and provided that the substitution results in a stable compound. A variety of locations may be substituted or the location may be specified. The functional group may include one or more atoms and may be added in one reaction or using several reactions.

The terms "stable compound" and "stable structure" refer to a compound that is sufficiently robust to survive isolation to a useful degree of purity from a reaction mixture and formulation into an efficacious agent.

The term "alkyl", as used herein, unless otherwise indicated, includes saturated monovalent hydrocarbon radicals having straight or branched moieties or combinations thereof. In some embodiments the alkyl has from 1-12 carbons. In some embodiments the alkyl has from 1-8 carbons. In some embodiments the alkyl has from 1-6 carbons. The term "lower alkyl" refers to an alkyl moiety containing from 1-4 carbon atoms.

The term "cycloalkyl" as employed herein, unless otherwise indicated, includes saturated cyclic hydrocarbon groups having from 3 to about 12 carbons. In some embodiments the cycloalkyl has from 3 to about 8 carbons. In some embodiments the cycloalkyl has from 3 to about 6 carbons. Cycloalkyl groups include, without limitation, cyclopropyl, cyclobutyl, cyclopentyl, cyclohexyl, cycloheptyl, and cyclooctyl.

The term "alkenyl", as used herein, unless otherwise indicated, includes unsaturated hydrocarbon radicals having straight or branched moieties or combinations thereof, with one or more double bonds. In some embodiments the alkenyl has one or two double bonds. In some embodiments the alkenyl has from 2-12 carbons. In some embodiments the alkenyl has from 2-8 carbons. In some embodiments the alkenyl has from 2-6 carbons.

The term "cycloalkenyl" as employed herein, unless otherwise indicated, includes partially unsaturated cyclic hydrocarbon groups having from 3 to about 12 carbons. In some embodiments the cycloalkenyl has from 3 to about 8 carbons. In some embodiments the cycloalkenyl has from 5 to about 6 carbons. Cycloalkenyl groups include, without limitation, cyclopentenyl and cyclohexenyl.

The term "aryl," as used herein, unless otherwise indicated, refers to phenyl or naphthyl optionally substituted with one or more substituents independently selected from halogen, (C₁-C₆)alkyl, (C₁-C₆)alkoxy, amino, (C₁-C₆)alkylamino, di-[(C₁-C₆)alkyl)]amino, nitro, cyano and trifluoromethyl. In some embodiments the aryl group is substituted with from zero to two substituents.

The term "acyl," as used herein, unless otherwise indicated, includes a radical of the general formula RCO wherein R is alkyl, alkoxy, aryl, arylalkyl, or arylalkyloxy and the terms "alkyl" or "aryl" are as defined above.

The term "acyloxy", as used herein, unless otherwise indicated, includes O-acyl groups wherein "acyl" is as defined above.

The term "heteroaryl", as used herein, unless otherwise indicated, refers to (C₂-C₉)heteroaryl containing one to five N, O or S atoms. In some embodiments the heteroaryl is a 5- or 6-membered heteroaryl. In some embodiments, the heteroaryl is selected from furyl, thienyl, thiazolyl, pyrazolyl, isothiazolyl, oxazolyl, isoxazolyl, pyrrolyl, triazolyl, tetrazolyl, imidazolyl, 1,3,5-oxadiazolyl, 1,2,4-oxadiazolyl, 1,2,3-oxadiazolyl, 1,3,5-thiadiazolyl, 1,2,3-thiadiazolyl, 1,2,4-thiadiazolyl, pyridyl, pyrimidinyl, pyrazinyl, pyridazinyl, 1,2,4-triazinyl, 1,2,3-triazinyl, 1,3,5-triazinyl, pyrazolo[3,4-b]pyridinyl, cinnolinyl, pteridinyl, purinyl, 6,7-dihydro-5H-[1]pyridinyl, benzo[b]thiophenyl, 5, 6, 7, 8-tetrahydro-quinolin-3-yl, benzoxazolyl, benzothiazolyl, benzisothiazolyl, benzisoxazolyl, benzimidazolyl, thianaphtenyl, isothianaphthenyl, benzofuranyl, isobenzofuranyl, isoindolyl, indolyl, indolinzinyl, indazolyl, isoquinolinyl, quinolinyl, phthalazinyl, quinoxalinyl,'quinazolinyl, and benzoxazinyl. One of ordinary skill in the art will understand that the connection of said (C₂-C₉)heteroaryl ring can be through a carbon atom or through a nitrogen heteroatom where possible.

The term "heterocycloalkyl," as used herein, refers to a (C₂-C₉) heterocycloalkyl containing one to five N, O or S atoms. In some embodiments the heterocycloalkyl is a 5- or 6-membered heterocycloalkyl. In a preferred embodiment, the heterocycloalkyl group is selected from pyrrolidinyl, tetrahydrofuranyl, dihydrofuranyl, tetrahydropyranyl, pyranyl, thiopyranyl, aziridinyl, oxiranyl, methylenedioxyl, chromenyl, isoxazolidinyl, 1,3-oxazolidin-3-yl, isothiazolidinyl, 1,3-thiazolidin-3-yl, 1-2-pyrazolidin-2-yl, 1,3-pyrazolidin-1-yl, piperidinyl, thiomorpholinyl, 1,2-tetrahydrothiazin-2-yl, 1,3-tetrahydrothiazin-3-yl, tetrahydrothiadiazinyl, morpholinyl, 1,2-tetrahydrodiazin-2-yl, 1,3-tetrahydrodiazin-1-yl, tetrahydroazepinyl, piperazinyl, and chromanyl. One of ordinary skill in the art will understand that the connection of the heterocycloalkyl ring can be through a carbon atom or through a nitrogen heteroatom where possible.

The term "one or more substituents", as used herein, refers to a number of substituents that equals from one to the maximum number of substituents possible based on the number of available bonding sites.

The term "halogen", as used herein, unless otherwise indicated, refers to chlorine, fluorine, bromine and iodine.

The term "effective" is used herein to indicate that the active agent is administered in an amount and at an interval that results in the desired treatment or improvement in the disorder or condition being treated (*e.g*., an amount effective to decrease pigmentation or ameliorate hyperpigmentation). This term is intended to include treatments for cosmetic purposes (e,g., cosmetically effective amounts). Similarly, the term "pharmaceutical" as used herein is intended to encompass cosmetic purposes.

### B) SKIN-LIGHTENING AGENTS

A useful active agent is a melanin-synthesis inhibitor. A nonlimiting example of a melanin-synthesis inhibitor is a tyrosinase inhibitor. Typically, the tyrosinase inhibitor will be 4-cyclopentyl-resorcinol.

4-cyclopentylresorcinol is of formula (X):

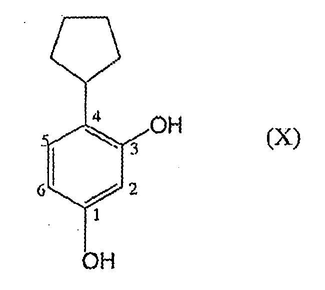

The resorcinol compound also include compounds identical to those described and depicted but for the fact that one or more hydrogen, carbon or other atoms are replaced by isotopes thereof. In some embodiments such compounds are useful as research and diagnostic tools in metabolism and pharmacokinetic studies and in binding assays.

Pharmaceutically acceptable acid and base addition salts of the resorcinol compound are also useful and can be made, *e.g*., as described in U.S. Patent No. 6,132,740 to Hu (issued October 17, 2000). The acids which are used to prepare the pharmaceutically acceptable acid addition salts of the aforementioned base compounds of this invention are those which form non-toxic acid addition salts. Nonlimiting examples of such salts include salts containing pharmacologically acceptable anions, such as the hydrochloride, hydrobromide, hydroiodide, nitrate, sulfate, bisulfate, phosphate, acid phosphate, acetate, lactate, citrate, acid citrate, tartrate, bitartrate, succinate, maleate, fumarate, gluconate, saccharate, benzoate, methanesulfonate, ethanesulfonate, benzenesulfonate, p-toluenesulfonate and pamoate (*i.e*., 1,1-methylene-bis-(2-hydroxy-3-naphthoate)) salts.

### C) FORMULATION

As noted above, the invention is directed to a formulation for delivering skin lightening agents. The formulation includes at least one skin-lightening agent and a carrier containing at least one hydroxyl solvent and at least one co-solvent. Typically, the formulation will be a liquid. It may be a solution, a suspension, or an emulsion, depending upon the specific components utilized and their relative concentration.

The concentration of the skin-lightening agent (i.e, the active agent) can vary widely. Typically, the active agent will be present in the formulation in the quantity of from about 0.1% (wt/vol) to about 10% (wt/vol). Alternatively, the amount of active agent is from about 0.1% (wt/vol) to about 5% (wt/vol) of the formulation. Alternatively, the amount of active agent in the formulation is from about 0.5 % (wt/vol) to about 4% (wt/vol) of the formulation. Alternatively, the amount of active agent in the formulation is from about 2% (wt/vol) to about 4% (wt/vol) of the formulation. Alternatively, the amount of active agent in the formulation is from about 1% (wt/vol) to about 3% (wt/vol) of the formulation. Alternatively, the amount of active agent in the formulation is from about 1% (wt/vol) to about 2% (wt/vol) of the formulation. By way of nonlimiting example, the amount of active agent in the formulation is about 1 % (wt/vol), about 2% (wt/vol), about 3% (wt/vol), or about 4% (wt/vol) of the formulation.

The formulation will also contain a carrier. The carrier may comprise up to 99.9% of the formulation. Typically, it will comprise at least 80% of the formulation. The quantity of carrier contained within the formulation will vary with the amount of skin lightening agent utilized and the presence of other active agents, which is described infra.

The carrier will contain at least one hydroxyl compound. The quantity of hydroxyl compound can vary, but will typically comprise from about 25% to about 95% (vol/vol) of the carrier. Typically the carrier will contain 85% (vol/vol) of the hydroxyl compound. Examples of hydroxyl compounds include glycols and lower alkanols. Admixtures of different hydroxyl compounds may be used.

The carrier comprises 45% (vol/vol) glycol.

The carrier comprises more than one glycol compound in order to balance the solubility of the components of the formulation. The carrier contains a mixture of hexylene glycol ("HG") and propylene glycol ("PG"). Hexylene glycol is also called 2-methyl-2, 4-pentanediol, and is available commercially, *e.g*., from Acros Organics (Morris Plains, NJ). Propylene glycol is also called 1,2-propanediol, and is available commercially, *e.g*., from Sigma (St. Louis, MO).

The carrier also comprises a lower alkanol which is ethanol. Lower alkanols can be commercially obtained, *e.g*., from Fisher Scientific (Pittsburgh, PA).

The carrier comprises 40% (vol/vol) lower alkanol.

The carrier will also contain one co-solvent. The co-solvent will be present in the quantity of about 15% (vol/vol).

The co-solvent is a dialkyl ether derivative of isosorbide, *e.g*., dimethyl isosorbide ("DMI"), which is available commercially, *e.g*., from Aldrich (Milwaukee, WI).

The co-solvent described above may contain chiral centers and therefore may exist in different enantiomeric and diastereomeric forms. Additionally, different regioisomers of the compound may exist. Any regioisomers, any optical isomers, and any stereoisomers of these compounds and mixtures thereof, can be used as the active agent in the formulation of the invention.

The ratio of the components in the carrier will vary depending on the solubility and permeability of the skin-lightening agent used in the formulation as well as other factors.

The formulation comprises: about 5% HG, about 40% PG, about 15% DMI, and about 40% ethanol (vol/vol/vol/vol).

In addition to the skin-lightening agent, one of skill in the art would be aware of numerous other pharmaceutical excipients that could be added to the carrier to enhance its elegance and appeal to the consumer. The CTFA Cosmetic Ingredient Handbook, Second Edition (1992) describes a wide variety of nonlimiting cosmetic and pharmaceutical ingredients commonly used in the skin care industry, which may be suitable for use in the formulation of the present invention. Nonlimiting examples of these ingredient classes include: aesthetic components such as fragrances, pigments, colorings/colorants, essential oils, skin sensates, astringents, etc. (*e.g*., clove oil, menthol, camphor, eucalyptus oil, eugenol, menthyl lactate, witch hazel distillate), anti-caking agents, antifoaming agents, antioxidants, binders, buffering agents, bulking figents, chelating agents, colorants, cosmetic astringents, cosmetic biocides, denaturants, pH adjusters, propellants, reducing agents, sequestrants, skin-conditioning agents (*e.g*., humectants, including miscellaneous and occlusive), skin soothing and/or healing agents (*e.g*., panthenol analogs (*e.g*., ethyl panthenol), aloe vera, pantothenic acid analogs, allantoin, bisabolol, and dipotassium glycyrrhizinate), etc.

Other active ingredients may be added to the formulation. One such example is sunscreens (UVA or UVB blockers). They may be incorporated into the formulation to prevent repigmentation, to protect against sun or UV-induced skin darkening or to enhance the ability to reduce skin melanin and the skin depigmentation action. *See, e.g.,* U.S. Patent No. 6,132,740 to Hu (issued October 17, 2000). The potential utility of incorporating other active ingredients beyond the skin-lightening agent will be readily apparent to one skilled in the art.

### D) METHOD OF MANUFACTURE

The formulation may be prepared using methods well known to those skilled in the art. For example, the formulation is prepared by mixing the carrier components together on a volume to volume or weight to weight basis. The carrier components are combined together in any order. The active agent is weighed out separately and combined with the carrier. In some embodiments, the carrier is added gradually to the dry active agent in order to slowly wet it. Alternatively, the active agent is added to the carrier, which can also be done gradually. In some embodiments, the active agent dissolves in the carrier. To facilitate dissolution, in some embodiments the container holding the carrier and the active agent are stirred by methods including but not limited to vortexing or placing a magnetic stir bar in the container which is placed on a magnetic stirrer.

### E) DOSAGE AND USE

In yet another aspect, the invention provides a method for lightening skin or preventing the formation of skin pigmentation. This method comprises administering to an area of a subject's skin an effective amount of a pharmaceutical formulation comprising a skin-lightening agent in an admixture with a pharmaceutically acceptable carrier. The formulation may be administered in one dose or may require multiple doses in order to achieve the desired effect.

In order to be effective, the appropriate dose regimen, the amount of each dose administered, and specific intervals between doses of the active compound will depend upon the particular active compound employed, the condition, size, and age of the patient being treated, the toxicity and half-life of the chosen active agent(s), the presence of other drugs in the patient, the effect desired, and the nature and severity of the disease, disorder or condition being treated. Specifically, the active compound is administered in an amount and at an interval that results in the desired treatment of or improvement in the disorder or condition being treated. The trial dosages would be chosen after consideration of the results of animal studies and the clinical literature. A physician would make these determinations.

For example, an effective dosage and treatment protocol can be determined by conventional means, starting with a low dose in laboratory animals and then increasing the dosage while monitoring the effects, and systematically varying the dosage regimen as well. Where possible, it is desirable first to determine *in vitro* the cytotoxicity of the compound to the tissue type to be treated, and then in a useful animal model system prior to testing and use in humans. Animal studies, such as mammalian studies, are commonly used to determine the maximal tolerable dose ("MTD") of a bioactive agent per kilogram weight. Those skilled in the art can extrapolate doses for efficacy and avoidance of toxicity to other species, including humans.

One of ordinary skill in the art will appreciate that the endpoint chosen in a particular case will vary according to the disease, condition, or disorder being treated, the outcome desired by the patient, subject, or treating physician, and other factors. When using the formulation to lighten skin color such as, for example, to reverse hyperpigmentation caused by, for example, inflammation or diseases such as melasma, any one of a number of endpoints can be chosen. Endpoints can be defined subjectively such as, for example, when the subject is simply "satisfied" with the results of the treatment. The endpoint can be determined by the satisfaction of the patient or the treating physician with the results of the treatment. Alternatively, endpoints can be defined objectively. For example, the patient's or subject's skin in the treated area can be compared to a color chart. Treatment can then be terminated when the color of the skin in the treated area is similar in appearance to a color on the chart. Alternatively, the reflectance of the treated skin can be measured, and treatment can be terminated when the treated skin attains a specified reflectance. Alternatively, the melanin content of the treated skin can be measured. Treatment can be terminated when the melanin content of the treated skin reaches a specified value. Melanin content can be determined in any way known to the art, including by histological methods, with or without enhancement by stains for melanin.

Typically however, the skin lightening agent will be present in the formulation in the quantity of 0.1 to 10% (wt/vol). The formulation will be applied to the skin requiring lightening from 1 to 4 times daily. Administration will continue on a daily basis until the desired pigmentation is achieved. Subsequent reapplications may be required on a periodic basis.

There are numerous conditions for which the skin-lightening formulation may be beneficial which would be known to one of skill in the art. *See, e.g.,* Freedberg et al., eds. (1999) Fitzpatrick's s Dermatology in General Medicine, Fifth Edition, 987, McGraw-Hill, New York. Such disorders include, but are not limited to, regional hyperpigmentation caused by melanocytic hyperactivity, such as idiopathic melasma occurring either during pregnancy (mask of pregnancy or chloasma) or secondary to estrogen-progesterone contraception; local hyperpigmentation caused by benign melanocytic hyperactivity and proliferation such as lentigo senilis; accidental hyperpigmentation such as post-lesional pigmentation and scarring; postinflammatory hyperpigmentation; and certain forms of leukoderma such as vitiligo where, if the injured skin cannot be repigmented, the residual zones of normal skin are depigmented to impart a homogeneous white color to the entire skin. In addition, there are numerous other conditions for which skin-lightening may be beneficial. By way of nonlimiting example, such skin conditions may be caused by lentigines, Moynahan's syndrome, centrofacial neurodysraphic lentiginosis, Peutz-Jegher syndrome, PUVA, Sotos' syndrome, solar lentigo (such as liver spots), eruptive lentigines, café au lait macules, neurofibromatosis, Albright's syndrome, Silver-Russell syndrome, Westerhof's syndrome, Watson's syndrome, Bloom's syndrome, gastrocutaneous syndrome, Becker's melanosis, nevus spilus, ephelides, NAME/LAMB syndrome, ichthyosis nigricans, porphyria cutanea tarda, hemochromatosis, hepatolenticular degeneration, Gaucher's disease, Niemann-Pick disease, ACTH- and MSH-producing tumors, exogenous ACTH therapy, Addison's disease, estrogen therapy, Carney's complex syndrome, arsenicals, busulfan, photochemical agents (such as psoralens or tar), Berloque dermatitis, Kwashiorkor, pellagra, sprue, vitamin B₁₂ deficiency, ultraviolet radiation (such as radiation tanning or suntanning), thermal radiation, alpha, beta, gamma ionizing radiation, trauma (such as chronic pruritus), postinflammatory melanosis (such as exanthems or drug eruptions), Lichen planus, discoid lupus erythematosus, melanoma, mastocytosis, acanthosis nigricans with adenocarcinoma and lymphoma, systemic scleroderma, chronic hepatic insufficiency, Whipple's syndrome, Cronkhite-Canada syndrome, neurocutaneous melanosis, familial periorbital hyperpigmentation, familial progressive hyperpigmentation, Dowling-Degos disease, dyskeratosis congenita, Fanconi's syndrome, human chimaera, acropigmentation of Dohi, reticulate acropigmentation of Kitamura, dermatopathia pigmentosa reticularis, POEMS syndrome, carbon baby syndrome, systemic 5-fluorouracil, cyclophosphamide, topical nitrogen mustard, Bleomycin, lichen simplex chronious, atopic dermatitis, psoriasis, and tinea versicolor.

In addition to pharmaceutical uses, the formulations of the current invention are useful for cosmetic purposes. Cosmetic applications for methods of the present invention include the topical application of compositions containing one or more compounds to enhance or otherwise alter the visual appearance of normal skin, which is not affected by a disorder. Occurrences in the skin of, noticeable but undesired pigmentation as a result of melanin production (*e.g*, freckles) can be treated using the methods of the present invention.

In still yet another aspect, the invention provides for a kit for preparing a skin-lightening formulation. The kit contains a skin-lightening agent, as described above, at least one container, a co-solvent, and at least one hydroxyl compound selected from the group consisting of glycols and lower alkanols. In some embodiments, the co-solvent and hydroxyl compound are premixed to form a carrier. In some embodiment, the carrier comprises a co-solvent, at least one glycol, and a lower alkanol. In certain particular embodiments, the carrier comprises an isosorbide, *e.g*., dimethyl isosorbide, at least one glycol, *e.g*., hexylene glycol and propylene glycol, and a lower alkanol, *e.g*., ethanol. The components of the carrier and the active arent(s) may or may not be provided in a preset unit dose or usage amount.

The container has a variety of purposes, including but not limited to preventing contamination, minimizing evaporation or drying, facilitating mixing, or a variety of other purposes known in the art. The container is made of any material suitable for the desired purpose. For example, in some embodiments the kit includes various containers for containing the components of the formulation and combining them, including but not limited to a divided bottle or a divided foil packet; however, in some embodiments the components are contained within a single, undivided container. Additional nonlimiting examples of containers include syringes, boxes, bags, and the like. For example, in some embodiments, the kit includes one containers containing a previouslyprepared formulation containing the skin-lightening agent as well as the carrier and any additional components as discussed above. Alternatively, the kit includes separate containers containing the skin-lightening agent and the carrier, and at least, one of the components of the carrier can be contained in another container. The components of the carrier can all be in separate containers or in containers containing one or more component, together as well. For example, the kit can include separate containers containing the skin-lightening agent and at least one of the co-solvent or the hydroxyl compound. Alternatively, the kit can include separate containers containing the skin-lightening agent and each of the co-solvent and the hydroxyl compound. The kit can also include additional containers in which to combine the various components of the formulation, including but not limited to, a container for combining the components of the carrier and a container for combining the carrier with the skin-lightening agent. The agent is mixed together with the carrier in a container. Alternatively, the components of the carrier are mixed together first and then mixed with the active agent. The kit can also include additional components, such as those described above. These additional components can be in separate containers and then mixed with the other components of the formulation. Alternatively, these additional components can be already mixed with one or more of the components of the formulation.

The same kit can be used for lightening skin and can also include an applicator for the skin-lightening formulation. As used herein, the term "applicator" means any instrument with which the formulation can be applied using any method of application. One of skill in the art would be aware of numerous instruments that could serve as applicators for topically administering the formulation. Nonlimiting examples of applicators include a sponge, a pipette, a cotton swab and a brush.

Optionally, the kit further contains a label indicating that the kit is to be used for lightening skin. The label may also contain instructions directing the procedure for combining the components if necessary and/or the use of such components for lightening skin. The kit may also optionally further contain a package insert indicating that the kit is to be used for lightening skin. The package insert may also optionally contain instructions directing the procedure for combining the components if necessary and/or the use of such components for lightening skin.

It may be desirable to provide a written memory aid, where the written memory aid is of the type containing information and/or instructions for the physician, pharmacist or subject. Nonlimiting examples of such a written memory aid include numbers on the containers corresponding with the days of the regimen on which the formulation should be applied, a card which contains the same type of information, or a calendar printed on a card *e.g*., as follows "First Week, Monday, Tuesday," ... etc .... "Second Week, Monday, Tuesday, ... " etc. Other variations of memory aids will be readily apparent.

The kit may also optionally include a dispenser designed to dispense the daily doses one at a time. A "daily dose" can be an application of the formulation or several applications of the formulation on a given day. In some cases, the dispenser is equipped with a memory-aid, so as to further facilitate compliance with the regimen. A nonlimiting example of such a memory-aid is a mechanical counter that indicates the number of daily doses that have been dispensed. Another nonlimiting example of such a memory-aid is a battery-powered micro-chip memory coupled with a liquid crystal readout, or audible reminder signal which, for example, reads out the date that the last daily dose has been administered and/or remands one when the next dose is to be administered.

The following nonlimiting examples further illustrate certain preferred embodiments of the present invention. The formulations were tested experimentally on Yucatan swine with dark skin. The Yucatan pigskin shares many physiologic and morphologic characteristic with human skin. For example, as described in U.S. Patent No. 5,470,567, the thickness and general morphology of epidermis and dermis, tritiated thymidine labeling pattern and index of epidermal cells, epidermal cell turnover time, as well as size, orientation, and distribution of vessels in skin are similar to that in humans. Varying concentrations of 4-cyclopentylresorcinol were administered to the back of Yucatan swine using various carriers. Representative data from the results of these experiments are shown in FIGS. 1-5.

### EXAMPLE 1: Yucatan Pig

### A. Animals

Yucatan swine with dark skin were used in this study. Swine were obtained from Charles River Laboratories, Windham, Me, and Sinclair Laboratories, MO.

### 13. Preparation of Skin-Lightening Agents and Formulation

4-cyclopentylresorcinol was synthesized and provided by Medicinal Chemistry OSIP/Birmingham, UK. All test materials were prepared by the Department of Pharmaceutical Development/OST Pharmaceuticals, Inc./Birmingham, UK and stored in the freezer at -20°C.

The solvents and other components used are commercially available and were obtained commercially from the following suppliers: ethanol (absolute) from Fisher (Pittsburgh, PA); propylene glycol (USP) from Sigma (St. Louis, MO); hexylene glycol from Acros Organics (Morris Plains, NJ); dimethyl isosorbide from Aldrich (Milwaukee, WI); and IPM from BUFA (Netherlands).

Test carriers, also called "vehicles" herein, that were combinations of differed solvent components were chosen based on the solubility of the skin-lightening agent in the individual solvents. Key factors when determining the test vehicle were the solubility of the skin-lightening agent in the vehicle, lack of precipitation of the skin-lightening agents in the vehicle, stability of the skin-lightening agent in the vehicle, and penetration of the skin by the skin-lightening agent in the vehicle.

A HG/PG/DMI/EtOH carrier was prepared by mixing together the four solvents on a volume to volume basis in the appropriate ratio. The solvents are mixed together in any order. To combine this carrier with the active agent, the carrier was gradually added to the dry active agent in order to slowly wet the dry material. The active agent then dissolved in the carrier by mixing.

Amounts of solution are measured by weight or volume. Either measurement can be used, but it is important to be consistent in the way the measurements are performed.

The solution of 1% 4-cyclopentylresorcinol in HG/PG/DMI/EtOH was prepared by weighing 0.40239 g of 4-cyclopentylresorcinol and adding 40 ml HG/PG/DMI/EtOH (5.1./2.2/15.4//19.5) (vol/vol/vol/vol) to yield 10.1 mg/ml of formulation solution.

Additional formulations with varying concentrations of active agent or other ingredients may be made as described above or as further described herein with modifications in the amounts used to produce formulations with the appropriate concentrations.

### C. Methods of Treatment and Evaluating Results

Seven sites measuring approximately 3 cm x 3 cm were marked on each side of the animal's spine, between the shoulder blade and hipbone. The sites were far enough apart so that there was no cross-over. Multiple treatments were performed on the back of each animal. Each animal in the experiment received the same treatments.

Each spot was topically treated with 20 µl of test solution applied twice daily, five days per week. This amounts to the same dose of active agent. Solutions were administered by pipetting the exact volume to the designated area with a micropipette and then spreading the solution. Each spot or test site on the animal's skin was graded 3 times a week on a scale of 0 to 4 for pigment change, erythema and scaling. Treatments were continued until pigmentation was reduced to a grade 1 level (marked uniform decrease in pigmentation) or for up to 1.2 weeks.

Results were analysed using different types of statistical analysis, ANOVA, Kruskal-Wallis test and Dunnett's multiple comparison, as indicated below. ANOVA is a statistical test that assumes sampled data is from populations that follow a Gaussian bell-shaped distribution and which works well, especially with large numbers, even if the distribution is only approximately Gaussian. The Kruskal-Wallis test is a nonparametric test that compares three or more unpaired groups. The Dunnett's test is a procedure for comparing each experimental mean with the control mean. For each graph presented, it is indicated which statistical tests were performed.

### D. Testing

The carriers, also called "vehicles" herein, were tested using two different skin-lightening agents. One vehicle tested was dimethyl isosorbide/hexylene glycol/propylene glycol/ethanol. The results are shown in Table 1 and in FIG. 1.

**Table 1**

| Active Agent | Test material concentration (% vol/vol) | Vehicle | Mean pigment Grade +/- SD¹ (Week 12) |
|---|---|---|---|
| | | DMI/HG/PG/EtOH² (15/5/40/35) | 4 +/- 0 |
| 4-cyclopentylresorcinol | 1% | DMI/HG/PG/EtOH² | 2.8 +/- 0.4* |

| | | | |
|---|---|---|---|
| *p<0.05, statistically different from the respective vehicles control using Kruskal-Wallis test. ¹graded on a scale of 0 to 4 in which 4 = normal skin color; 3 =slight decrease in pigmentation: 2 = definite decrease in pigmentation; 1 = marked uniform decrease in pigmentation; and 0 = complete depigmentation. ²Dimethyl isosorbide/hexylene glycol/propylene glycol/ethanol | | | |

FIG. 1 compares the depigmenting effect on Yucatan swine of topically applied 1% (wt/vol) 4-cyclopentylresorcinol, in HG/PG/DMI/EtOH, and the HG/PG/DM1/EtOH solution alone. This figure shows that the active agent has increased efficacy in HG/DMI/PG/EtOH. The data regarding the use of this skin-lightening agent in PG/EtOH is presented in Table 2 below.

No evidence of local skin irritation was apparent over any of the treated sites.

### E. Repigmentation

Pigmentation was monitored for 6 weeks after discontinuation of treatment following 12 weeks of topical treatment with 1% 4-cyclopentylresorcinol in HG/DMI/PG/EtOH (5/15/40/40).

FIG. 2 compares the reversibility of depigmentation after treatment discontinuation for the HG/PG/DMI/EtOH solution alone to treatment with different percentages of 4-cyclopentylresorcinol in the solution. The results show that the test sites treated with 1% 4-cyclopentylresorcinol show signs of gradual repigmentation during 6 weeks after discontinuation, of treatment. Thus, the depigmenting effect was not permanent.

### F. Collated Results

The results from several sets of experiments were collated to provide a more comprehensive comparison of the efficacy of 4-cyclopentylresorcinol in different vehicles. Additional vehicles tested that are included in this compilation include: 1) propylene glycol/ethanol (PG/EtQH), which was prepared on a volume to volume basis, to which the active agent was added on a weight to volume basis and vortexed until in solution, and 2) HG/PC/DMI/EtOH + 2% (wt/wt) isopropyl myristate ("IPM"), which was prepared by combining HG/PG/DMI/EtOH/IPM in a ratio of 5/40/15/36/2 (wt/wt/wt/wt/wt) to which the active agent was added on a weight to volume basis. IPM was added as a penetration enhancer to determine whether it increased the efficacy of the formulation. The compiled results are presented in Table 2.

**Table 2**

| | **Active Agent (mean pigment grade +/- SD).^{1,2}** | |
|---|---|---|
| **Vehicle** | **1%4-cyclopentyl resorcinol** | **2%4-cyclopentylresorcinol** |
| PG/EtOH(3/7) | 3.3+.-0.7 | 2.4+-/-0.9* |
| | (12) | (18) |
| HG/PG/DMI/EtOH | 2.8 +/- 0.6* | 2.0 +/-0.7* |
| (5/40/15/40) | (10) | (5). |
| HG/PG/DMI/EtOH+ | 2.0+/-1.2* | 1.4+/-0.9* |
| 2% IPM | (5) | (5) |

| | | |
|---|---|---|
| ¹Graded on a scale of 0 to 4, 0 = complete depigmentation, 1 marked even reduction in pigmentation, 2 = moderate even reduction in pigmentation, 3 slight uneven reduction in pigmentation and 4 = normal skin color ²Numbers in () show number of animals used *p< 0,05, statistically different, from the respective vehicle systems which showed no evidence of depigmentation (grade = 4.0) ³Vehicle was PG/EtOH (5/5) | | |

The Kruskall-Wallis Test and Dunnetis multiple comparison test were used to calculate these results based on the compilation of data from several different experiments using the same protocol.

These formulations were all prepared substantially as described above; however, the amounts were modified to produce the appropriate concentration.

FIG. 3 shows the comparative effect of topically applied 4-cyclopentylresorcinol on Yucatan swine pigmentation in three different vehicle systems. The data was analyze using a nonparametric method (the Kruskal-Wallis test and Dunnett's multiple comparison).

FIG. 3A compares the effect on the skin pigmentation of Yucatan swine of topical administration of 1% (wt/vol) and 2% (wt/vol) 4-cyclopentylresorcinol in PG/EtOH (30/70) to vehicle alone.

Fig. 3B shows a comparison of 4-cyclopentylresorcinol in HG/PG/DMI/EtOH (5/40/15/40) at 1% (wt/vol) and 2% (wt/vol) concentrations compared to 2% (wt/vol) 4. cyclopentylresorcinol in PG/EtOH and to the HG/PG/DMI/EtOH vehicle alone.

FIG. 3C shows the effects of adding 2% (wt/wt) IPM to the HG/PG/DMI/EtOH vehicle. FIG. 4C also compares the effect on the skin pigmentation of Yucatan swine of topical administration of 1% (wt/vol) and 2% (wt/vol) 4-cyclopentylresorcinol in HG/PG/DMI/EtOH/IPM to 2% (wt/vol) 4-cyclopentylresorcinol in PG/EtOH and to the HG/PG/DMI/EtOH plus 2% (wt/wt) IPM vehicle alone.

The results show that in HG/PG/DMI/EtOH, 1% and 2% 4-cyclopentylresorcinol are statistically different from vehicle and show a trend in the direction of reduced pigmentation but arc not statistically different from the results using the PG/EtOH vehicle. No irritation was apparent.

FIG. 4 shows a comparison of the effect of the topical administration of 4-cyclopentylresorcinol in two different vehicles on skin pigmentation in Yucatan swine. The data was analyzed using a nonparametric method (the Kruskal-Wallis test and Dunnett's multiple comparison).

FIG. 4A compares the effect on the skin pigmentation of 1% (wt/vol) and 2% (wt/vol) 4-cyclopentylresorcinol in an HG/PG/DMI/EtOH/IPM vehicle to the HG/PG/DMI/EtOH plus 2% (wt/wt) IPM vehicle alone.

FIG. 4B compares the effect on the skin pigmentation of 1% (wt/vol) and 2% (wt/voi) 4-cyclopentylresorcinol in a HG/PG/DMI/EtOH vehicle to the HG/PG/DM/EtOH vehicle alone. No irritation was apparent. Overall, the results in these Figures indicate that the HG/PG/DMI/EtOH vehicle improved the depigmentation efficacy of 4-cyclopentylresorcinol in Yucatan pigs. No irritation was observed for those formulations using HG/PG/DMI/EtOH as a vehicle.

### EQUIVALENTS

While the foregoing invention has been described in some detail for purposes of clarity and understanding, it will be appreciated by one shilled in the art from a reading of this disclosure that various changes in form and detail can be made without departing from the scope of the invention and the attached claims.

## Claims

1. A topical formulation comprising:
a) from 0.1 to 10 w/v% of 4-cyclopentyl resorcinol,
b) 5 v/v% of hexylene glycol,
c) 40 v/v% of propylene glycol
d) 15 v/v% of dimethylisosorbide, and,
e) 40 v/v% of ethanol.

2. The formulation of claim 1 which contains isopropyl myristate.

3. Use of a topical formulation according to claim 1 for the manufacture of a medicament for lightening skin.

4. A cosmetic method for lightening skin, comprising applying the topical formulation of claim 1 to the area of the skin requiring lightening.

## Patentansprüche

1. Topische Formulierung, umfassend:
a) 0,1 bis 10 G/V-% 4-Cyclopentylresorcin,
b) 5 V/V-% Hexylenglykol,
c) 40 V/V-% Propylenglykol,
d) 15 V/V-% Dimethylisosorbid und
e) 40 V/V-% Ethanol.

2. Formulierung gemäß Anspruch 1, die Isopropylmyristat enthält.

3. Verwendung einer topischen Formulierung gemäß Anspruch 1 für die Herstellung eines Medikaments zum Aufhellen der Haut.

4. Kosmetisches Verfahren zum Aufhellen der Haut, umfassend Auftragen der topischen Formulierung gemäß Anspruch 1 auf den Bereich der Haut, der ein Aufhellen erfordert.

## Revendications

1. Formulation topique comprenant :
a) de 0,1 à 10 % en poids/volume de 4-cyclopentyl-résorcinol,
b) 5 % en v/v d'hexylène glycol,
c) 40 % en v/v de propylène glycol,
d) 15 % en v/v de diméthylisosorbide et
e) 40 % en v/v d'éthanol.

2. Formulation selon la revendication 1, qui contient du myristate d'isopropyle.

3. Utilisation d'une formulation topique selon la revendication 1 pour la fabrication d'un médicament pour éclaircir la peau.

4. Procédé cosmétique pour éclaircir la peau, comprenant l'application de la formulation topique selon la revendication 1 à la zone de la peau nécessitant un éclaircissement.
